# EUROPEAN PATENT SPECIFICATION

(11) **EP 4 223 208 B1**
(45) Date of publication and mention of the grant of the patent: **29.07.2026**
(21) Application number: 22155415.7
(22) Date of filing: 07.02.2022
(51) Int. Cl.: A61B 1/005, A61B 1/00

(54) **AN ENDOSCOPE**
ENDOSKOP
ENDOSCOPE

(43) Date of publication of application: 09.08.2023
(73) Proprietor: Ambu A/S, 2750 Ballerup (DK)
(72) Inventor: QUENSEL, Ulf, 271 33 Ystad (SE); TORNAGHI, Barbara, 20900 Monza (MB) (IT); BESANA, Andrea, 20822 Seveso (MB) (IT); GRAW, Felix Andreas, 86152 Augsburg (DE)
(74) Representative: AWA Denmark A/S

(56) References cited:
- US-A1- 2005 070 885
- US-A1- 2013 012 781
- US-A1- 2017 086 651
- US-A1- 2017 172 386

## Description

The present disclosure relates to insertion endoscopes, in particular but not limited to, duodenoscopes.

Insertion endoscopes typically comprise a handle at the proximal end to be gripped by an operator and a flexible elongated insertion cord terminated at the distal end in a tip part at the end of a highly bendable, e.g. articulated, bending section, controllable by the operator. The tip part normally comprises a visual inspection means such as a camera, and illumination means such as LED's or exit apertures of light fibres and whatever optics are needed in that connection as well as tools or tool lifters. Electrical wiring for the camera and other electronics such as the LED lighting as well as pull wires run along the inside of the elongated insertion cord from the handle to the tip at the distal end. When, as mentioned, the illumination is instead fibre-optic, the optical fibres also run along inside of the elongated insertion cord.

Thus, the controllable bending section is normally an articulated section at the distal tip of the elongated insertion cord that can be controlled by the operator via control knobs arranged on the handle. Typically, this control is effected by tensioning or slacking the pull wires also running along the inside of the elongated insertion cord from the tip part to a control mechanism of the handle. Furthermore, a working channel may run along the inside of the elongated insertion cord from the handle to the tip, e.g. allowing liquid to be removed from the body cavity or allowing the insertion of surgical instruments or the like into the body cavity.

Thus, using the controls allows the operator to advance the distal tip of the endoscope to a desired location by means of a series of actions involving *inter alia* bending the bending section in a desired direction, advancing the elongated insertion cord and turning the elongated insertion cord by turning the handle which is rigidly connected thereto. Navigating a tortuous path of bends and turns to a location of interest may subject the elongated insertion cord including the distal controllable bending section to substantial forces including compression, torsion, and bending. The main body of the elongated insertion cord is essentially only bendable enough to follow the direction taken by the bending section. In fact, it could be said that it is an essential part of the purpose of the elongated insertion cord to transmit the longitudinal pushing forces and rotary torsional forces from the handle to the distal end of the elongated insertion cord in order to allow these maneuvers.

*Inter alia* to display images from the tip to allow the operator to find the way through the insertion maneuvers as well as during any procedure at a target location, the endoscope is connected to a display device which may also include other functionalities such as power supply or supply of light through the light guides, all depending on the actual type and construction of the endoscope. This connection is normally referred to as an umbilical.

In prior art the umbilical typically extends from the proximal end of the handle in a cross-wise direction as compared to the overall length of the endoscope, i.e. perpendicular to the longitudinal axis of the insertion cord when the insertion cord is in an unbent state extending straight as a natural extension of the handle towards the distal end of the endoscope. When gripping the handle with one hand and holding the endoscope with the insertion cord pointing downward from the handle, as e.g. illustrated in figure 18 of JP-2006-149880, the umbilical rests on the gripping hand extending backwards along the arm of the hand and past the side of the operator's body and so aids in supporting and balancing the endoscope. This in turn allows the operator to somewhat loosen the grip on the handle without losing control of the endoscope. This aid in support and balance may also exist in many situations throughout procedures, even if, through the maneuvers, the endoscope ends up in orientations that are not vertical or even far from vertical.

The ability to loosen the grip during procedures is of importance to the operator as the procedure may take an hour or even more and the position of the handle may be awkward after insertion of the insertion cord into the patient, and it may stressful or even impossible to maintain a firm grip throughout the procedure. JP-2006-149880, also discloses a handle where a detachable strap is provided. This detachable strap would seem to engage the back of the hand, possibly allowing the operator to loosen the grip. The balance would however still be influenced by the umbilical extending backward over the hand and arm. Furthermore, the strap though detachable allows only for one configuration and does not allow the operator to alter the grip.

However, for other reasons it may be desirable to have the umbilical extend elsewhere from the endoscope than perpendicular to the proximal end of the handle. This, on the other hand removes the support aid from the umbilical and changes the balance of the handle and another way of allowing the operator to loosen the grip must be devised. In this respect US2006/0287575 discloses an endoscope where the umbilical is connected to the endoscope at a location in the proximity of the tool entry port on the transition section of the handle between the gripping section and the insertion cord. A fixed hook is arranged in front of the part of the gripping section where the operator's fingers (apart from the thumb) engage the handle and buttons and prevents the operator from dropping the handle if the grip is loose.

It can also be envisaged that the endoscope is self-contained as far as power supply etc. is concerned and communication is wireless so that an umbilical is not needed at all. In this respect US5152278 discloses an endoscope with a hook similar to that of US2006/0287575 but in an endoscope with entirely different ergonomics and balance as compared to screen monitoring. Document US 2013/012781 A1 discloses an endoscope comprising a handle with an operation section main body. The operation section includes operating members in the form of a vertical lever and lateral lever arranged on side surfaces of the operation section main body for bending operations. An engage lever is provided as a braking operation body, with one end portion and the other end portion pivotally supported at predetermined positions on both left and right side surfaces of the operation section main body.

Based on this it is the object of the present disclosure to provide an endoscope with a handle with good ergonomics allowing the operator to loosen the grip around the handle during a procedure without relying on the support and balancing from the umbilical.

According to a first aspect of the disclosure this object is achieved by an endoscope comprising at the proximal end a handle adapted to be gripped by a hand of an operator, the handle comprising a proximal operating section, a distal transition section from which an insertion cord extends towards the distal end of the endoscope, an intermediate gripping section adapted to be gripped by palm and fingers of said hand of the operator, and a wing extending from the operation section,
wherein the wing is selectively movable between various positions with respect to the handle.

Hereby a versatile solution is achieved in that the position of the wing can be moved to meet operator needs or preferences. Some operators may prefer to have the wing in one position during a certain part of the procedure, and then move the wing to another position during another part of the procedure.

According to a second aspect of the disclosure the object is achieved by a system comprising a display device and an endoscope according to the first aspect connectable to said display device.

According to an embodiment of the first aspect of the disclosure, the wing is movable through a rotary motion path about a wing rotation axis.

According to an embodiment of the first aspect of the disclosure, the length of the wing is in the range between 40 mm and 100 mm preferably between 50 mm and 85 mm. Lengths in this interval have been found to cover most hand sizes of operators, while still being comfortable for all.

According to an embodiment of the first aspect of the disclosure, the insertion cord defines a longitudinal axis and said rotation axis is perpendicular to said longitudinal axis of the insertion cord.

According to an embodiment of the first aspect of the disclosure, the operating section comprises at least one rotary operating member rotatable about an operating member axis.

According to an embodiment of the first aspect of the disclosure, the rotary operating member is located on an opposite side of the handle as seen from the wing.

According to an embodiment of the first aspect of the disclosure, the wing rotation axis coincides with the operating member axis.

According to an embodiment of the first aspect of the disclosure, at least one predetermined arresting position is provided along the rotary motion path.

According to an embodiment of the first aspect of the disclosure, the tip of the wing is resiliently deflectable away from the handle. It has been found to increase comfort because it allows the back of the hand to be supported while at the same time the resilient deflection keeps the localized pressure on the back of the hand down.

According to an embodiment of the first aspect of the disclosure the wing comprises a core member with a Shore A hardness of or exceeding 80. This has been found to achieve a comfortable pressure on the back of the hand. This is in particular the case if, according to a further embodiment of the first aspect of the disclosure, the core member is covered by a softer coating. Accordingly, in an embodiment of the first aspect of the disclosure, this softer coating has a Shore A hardness in the range from 40 to 60, preferably approximately 50.

The disclosure will now be made in greater detail based on nonlimiting exemplary embodiments and with reference to the schematic drawings on which:
Fig. 1 shows a system of a display unit and a prior art endoscope connected to the display unit via an umbilical,
Fig. 2 shows a front view of an endoscope according to the disclosure, and
Fig 3 shows a left-hand side view of the endoscope according to the disclosure.

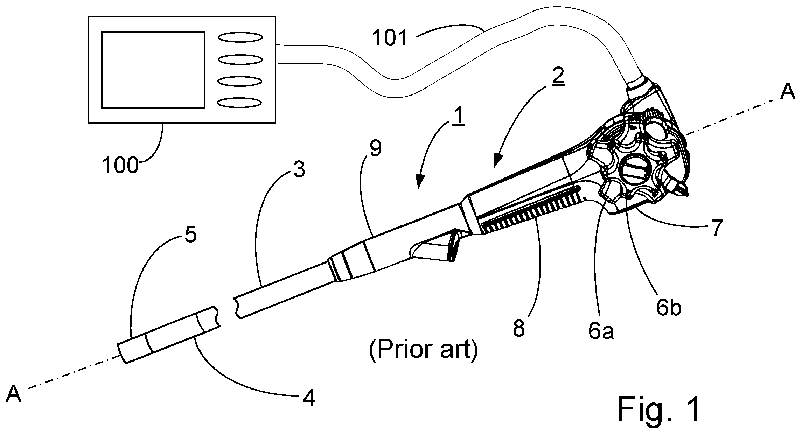

Turning first to Fig. 1 a prior art endoscope 1 is shown. The endoscope 1 is part of a system also comprising a display device 100 to which the endoscope 1 may be connected using an umbilical 101 as shown. The endoscope comprises a handle 2 forming the proximal end of the endoscope and an insertion cord 3 extending from the handle towards the distal end of the endoscope 1. The endoscope comprises a highly flexible bending section 4 terminating in a tip housing 5 at the distal end of the endoscope 1. The bending section 4 may be controlled by an operator using one or more rotary operating members 6a, 6b such as wheels or turn knobs arranged at an operating section 7 forming the most proximal part of the handle 2. The handle furthermore comprises a gripping section 8 adapted to be gripped by a hand of an operator. The handle 2 furthermore comprises a transition section 9 from which the insertion cord 3 extends. The gripping section is thus located between the operating section and the transition section as seen in the direction along the longitudinal axis A-A of the endoscope 1. The longitudinal axis is defined by the centre axis of the insertion cord 3 in a straight unbent state. This definition applies to the remainder of the present disclosure too. As will be understood the handle 2 also has an elongate shape generally aligned with the centre axis of the insertion cord 3 so that at least some parts of the handle 2, e.g. of the transition section 9, exhibit rotational symmetry about the longitudinal axis A-A. As will be noted the umbilical 101 extends in a cross-wise direction to the longitudinal axis A-A, but with an off-set, i.e. not intersecting the longitudinal axis A-A. In the illustrated prior art example of Fig. 1 the umbilical 101 extends backward from endoscope handle 2 at the left-hand side thereof. As to the references up, down, left, right, front back the presumption is that the endoscope 1 is held in front of the operator standing with the lower left arm straight and gripping the gripping section 8 of the endoscope, and the insertion cord 3 pointing vertically down, i.e. that the axis A-A is vertical. The references up, down, left, right, front back as applied to the endoscope 1 are then used in the same manner as they would normally apply to the standing operator. This convention applies throughout the disclosure.

Apart from the existence and location of the umbilical the above explanation of details of the endoscope 1, the display unit 100, and the system they form may also apply to the following disclosure and accordingly same reference numerals will be used for same or corresponding parts.

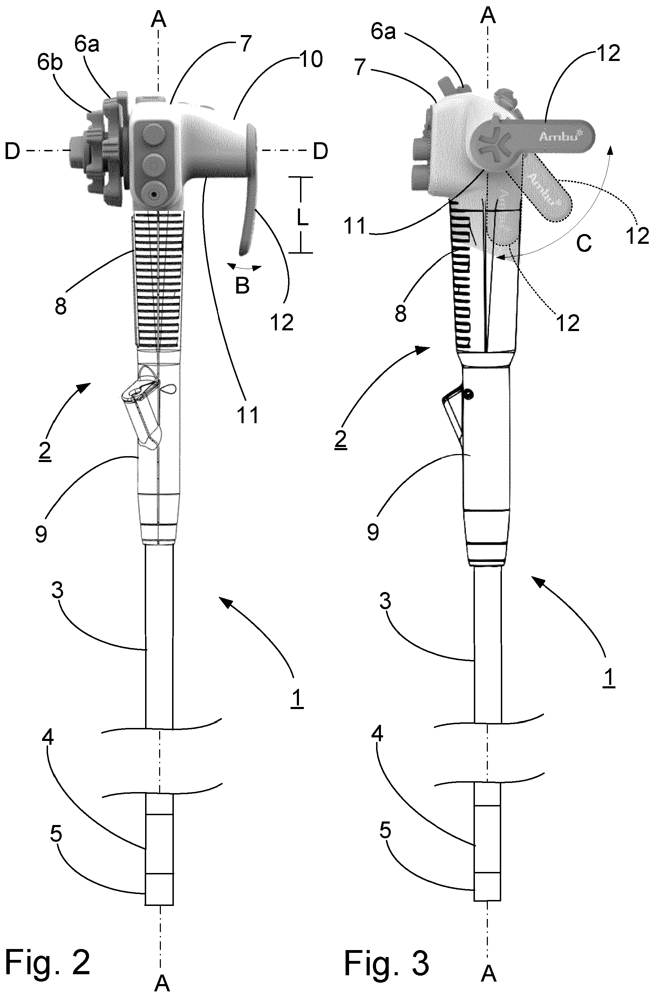

Turning now to Fig. 2 an endoscope according to the present disclosure is shown in front view. An umbilical 101 is not shown, as it may extend from many various places on the endoscope 1 or may even be entirely omitted if the endoscope 1 is self-contained in terms of power supply etc. and communication to the display unit 100 is wireless.

As can be seen the operating section 7 comprises a cross-wise protrusion 10 on the side opposite the operating members 6a, 6b, i.e. the left-hand side in the embodiment shown. The protrusion 10 has a rounded lower surface adapted to rest on the web between the operator's thumb and index finger of the left hand. The remaining fingers and possibly the index finger will curve around the gripping section 8 to the front in order to grip the handle 2. Connected to the protrusion is a wing 12. The wing 12 is adapted to engage the back of the hand gripping the gripping section 8, and so forms a hook preventing the palm of the hand from moving too far away from the gripping section 8 if the operator loosens the grip e.g. by straightening the curved gripping fingers. This is in particular relevant if the handle 2 is not in the shown vertical position but has to be held in e.g. a horizontal position with the right-hand side down over a longer period during a procedure, where the operator might need to relax the gripping fingers. The wing 12 preferably has some lateral resilient flexibility as indicated with the double arrow B in order not to press too hard on the back of the operator's hand, and may furthermore have a soft comfortable surface, i.e. softer than the material from which the handle 2 or at least the gripping section 8 thereof is made. If the wing 12 or at least a core member thereof is made from a material with a Shore A hardness of or exceeding 80, e.g. a hard silicone material, the desired resiliency may be provided. The core could also comprise the same material as the handle 2, e.g. PP and/or ABS. On the more hard and rigid core, providing the resilient flexibility of the wing 12, a softer more comfortable outer layer may be provided. This softer more comfortable layer could also comprise silicone, albeit with a lower Shore A hardness in the range from 40 to 60, preferably approximately 50.

The length L of the wing 12, i.e. the part extending over the protrusion 10, is in the range between 40 mm and 100 mm preferably between 50 mm and 85 mm. This has been found to cover and be comfortable and yield effective support for most operators' hands, be it of a bigger man with a hand width of e.g. 105 mm or a smaller woman with a hand width of e.g. 65 mm.

The horizontal position described above is, however, not the only awkward position in which the handle 2 may need to be held during a procedure.

This is solved by the fact that the wing 12 is not fixed as a clip on the handle 2 is movable through a rotary motion path as indicted by the double arrow C in Fig. 3 about a wing rotation axis D-D as seen in Fig. 2. That is to say, from the vertical position shown in Fig. 2 in dashed lines through a number of positions to horizontal as shown in full on Fig. 3.

The wing rotation axis D-D is preferably perpendicular to the longitudinal axis A-A of the endoscope. The wing rotation axis D-D may intersect the longitudinal axis A-A or it may have a slight off-set. The wing rotation D-D axis is preferably parallel to an operating member axis, i.e. the rotary axis of the rotary operating member 6a and/or rotary operating member 6b. They may even coincide as in the embodiment illustrated. Irrespectively, of whether the axes are parallel or even coincide, it is preferred that the rotary operating member 6a, 6b is located on an opposite side of the handle 2 as seen from the wing 12 to provide good access to the rotary members 6a, 6b for the operator's right hand without being hindered by the wing 12.

As mentioned, the wing 12 is movable through a number of positions, this may be an infinite number, where the wing 12 held in a position chosen at will by the operator and held there, e.g. by friction between the wing 12 and suitable parts of the handle. The force to be applied to the tip of the wing 12 in order to overcome friction and turn the wing 12 should preferably be in the range of 10-15 N in order to prevent the operator form inadvertently rotating the wing 12 during procedure.

There may also or instead be a finite number of predetermined arresting positions provided along the rotary motion path C, e.g. where protrusions on the wing 12 elastically click into matching recesses provided in handle 2, or vice versa. The force to be applied to the tip of the wing 12 for the wing 12 to leave these arresting positions should preferably exceed the 10-15 N mentioned above. In all cases the movable wing 12 allows the operator to adapt the position of the wing 12 with respect to the handle 2, so that is allows for support of the handle in many different orientations, in turn allowing the operator to relax the grip during extended procedures.

It should be noted that although the angle of the rotary path C is shown as 90° between vertical and horizontal other angles could be envisaged. This is just one variant among the numerous modifications the skilled person will identify to the exemplary embodiment described without departing from the scope of the claims.

## Claims

1. An endoscope (1) comprising at the proximal end a handle (2) adapted to be gripped by a hand of an operator, the handle (2) comprising a proximal operating section (7) having operating members (6a, 6b) and comprising a protrusion (10) on the side of the proximal operating section (7) opposite the operating members (6a, 6b), a distal transition section (9) from which an insertion cord (3) extends towards the distal end of the endoscope (1), and an intermediate gripping section (8) adapted to be gripped by palm and fingers of said hand of the operator, wherein
a wing is (12) connected to the cross-wise protrusion (10) and extending from the operation section (7),
the wing (12) being selectively movable between various positions with respect to the handle (2).

2. An endoscope (1) according to claim 1, wherein the wing (12) is movable through a rotary motion path (C) about a wing rotation axis (D-D).

3. An endoscope (1) according to claim 2, wherein the insertion cord (3) defines a longitudinal axis (A-A) and said rotation axis (D-D) is perpendicular to said longitudinal axis (A-A) of the insertion cord (3).

4. An endoscope (1) according to any one of the preceding claims wherein the operating section (7) comprises at least one rotary operating member (6a, 6b) rotatable about an operating member axis.

5. An endoscope (1) according to any one of claims 3 or 4, wherein the wing rotation axis (D-D) coincides with the operating member axis.

6. An endoscope (1) according to any one of the preceding claims wherein the length of the wing (12) is in the range between 40 mm and 100 mm preferably between 50 mm and 85 mm.

7. An endoscope (1) according to any one of claims 2 to 6, wherein at least one predetermined arresting position for the wing (12) is provided along the rotary motion path (C).

8. An endoscope (1) according to any one the preceding claims wherein the tip of the wing (12) is resiliently deflectable away from the handle (2).

9. An endoscope (1) according to any one of the preceding claims, wherein the wing (12) comprises a core member with a Shore A hardness of or exceeding 80.

10. An endoscope (1) according to claim 9, where in the core member is covered by a softer coating.

11. An endoscope (1) according to claim 10, wherein the coating has a Shore A hardness in the range from 40 to 60, preferably approximately 50.

12. A system comprising a display device (100) and an endoscope (1) according to any one of the preceding claims connectable to said display device (100).

## Patentansprüche

1. Endoskop (1), umfassend einen Griff (2) an dem proximalen Ende, der dazu ausgeführt ist, von einer Hand einer Betätigungsperson ergriffen zu werden, wobei der Griff (2) einen proximalen Betätigungsabschnitt (7), der Betätigungsglieder (6a, 6b) aufweist und einen Vorsprung (10) an der den Betätigungsgliedern (6a, 6b) gegenüberliegenden Seite des proximalen Betätigungsabschnitts (7) umfasst, einen distalen Übergangsabschnitt (9), von dem sich ein Einführschlauch (3) zu dem distalen Ende des Endoskops (1) hin erstreckt, und einen Zwischengreifabschnitt (8) umfasst, der dazu ausgeführt ist, von der Handfläche und den Fingern der Hand der Bedienperson ergriffen werden kann, wobei
ein Flügel (12) mit dem Quervorsprung (10) verbunden ist und sich von dem Betätigungsabschnitt (7) erstreckt,
wobei der Flügel (12) gezielt zwischen verschiedenen Positionen bezüglich des Griffs (2) bewegbar ist.

2. Endoskop (1) nach Anspruch 1, wobei der Flügel (12) durch einen Drehbewegungsweg (C) um eine Flügeldrehachse (D-D) beweglich ist.

3. Endoskop (1) nach Anspruch 2, wobei der Einführschlauch (3) eine Längsachse (A-A) definiert und die Drehachse (D-D) senkrecht zu der Längsachse (A-A) des Einführschlauchs (3) verläuft.

4. Endoskop (1) nach einem der vorhergehenden Ansprüche, wobei der Betätigungsabschnitt (7) mindestens ein Drehbetätigungsglied (6a, 6b) umfasst, das um eine Betätigungsgliedachse drehbar ist.

5. Endoskop (1) nach einem der Ansprüche 3 oder 4, wobei die Flügeldrehachse (D-D) mit der Betätigungsgliedachse zusammenfällt.

6. Endoskop (1) nach einem der vorhergehenden Ansprüche, wobei die Länge des Flügels (12) im Bereich zwischen 40 mm und 100 mm, vorzugsweise zwischen 50 mm und 85 mm, liegt.

7. Endoskop (1) nach einem der Ansprüche 2 bis 6, wobei mindestens eine vorbestimmte Arretierposition für den Flügel (12) entlang des Drehbewegungswegs (C) bereitgestellt ist.

8. Endoskop (1) nach einem der vorhergehenden Ansprüche, wobei die Spitze des Flügels (12) elastisch von dem Griff (2) weg auslenkbar ist.

9. Endoskop (1) nach einem der vorhergehenden Ansprüche, wobei der Flügel (12) ein Kernelement mit einer Shore-A-Härte gleich oder größer 80 umfasst.

10. Endoskop (1) nach Anspruch 9, wobei das Kernelement mit einer weicheren Beschichtung überzogen ist.

11. Endoskop (1) nach Anspruch 10, wobei die Beschichtung eine Shore-A-Härte im Bereich von 40 bis 60, vorzugsweise etwa 50 aufweist.

12. System, umfassend eine Anzeigevorrichtung (100) und ein Endoskop (1) nach einem der vorhergehenden Ansprüche, das mit der Anzeigevorrichtung (100) verbindbar ist.

## Revendications

1. Endoscope (1) comprenant au niveau de l'extrémité proximale une poignée (2) conçue pour être saisie par une main d'un opérateur, la poignée (2) comprenant une section de fonctionnement (7) proximale ayant des éléments de fonctionnement (6a, 6b) et comprenant une protubérance (10) sur le côté de la section de fonctionnement (7) proximale en regard des éléments de fonctionnement (6a, 6b), une section de transition distale (9) à partir de laquelle un cordon d'insertion (3) s'étend vers l'extrémité distale de l'endoscope (1), et une section de préhension intermédiaire (8) conçue pour être saisie par la paume et les doigts de ladite main de l'opérateur, dans lequel une aile (12) est reliée à la protubérance transversale (10) et s'étend à partir de la section de fonctionnement (7),
l'aile (12) pouvant être déplacée sélectivement entre différentes positions par rapport à la poignée (2).

2. Endoscope (1) selon la revendication 1, dans lequel l'aile (12) est mobile selon un trajet de mouvement rotatif (C) autour d'un axe de rotation d'aile (D-D).

3. Endoscope (1) selon la revendication 2, dans lequel le cordon d'insertion (3) définit un axe longitudinal (A-A) et ledit axe de rotation (D-D) est perpendiculaire audit axe longitudinal (A-A) du cordon d'insertion (3).

4. Endoscope (1) selon l'une quelconque des revendications précédentes, dans lequel la section de fonctionnement (7) comprend au moins un élément de fonctionnement rotatif (6a, 6b) pouvant tourner autour d'un axe de l'élément de fonctionnement.

5. Endoscope (1) selon l'une quelconque des revendications 3 ou 4, dans lequel l'axe de rotation d'aile (D-D) coïncide avec l'axe d'élément de fonctionnement.

6. Endoscope (1) selon l'une quelconque des revendications précédentes, dans lequel la longueur de l'aile (12) est dans la plage entre 40 mm et 100 mm, de préférence entre 50 mm et 85 mm.

7. Endoscope (1) selon l'une quelconque des revendications 2 à 6, dans lequel au moins une position d'arrêt prédéterminée pour l'aile (12) est disposée le long du trajet de mouvement rotatif (C).

8. Endoscope (1) selon l'une quelconque des revendications précédentes, dans lequel la pointe de l'aile (12) peut être déviée élastiquement à distance de la poignée (2).

9. Endoscope (1) selon l'une quelconque des revendications précédentes, dans lequel l'aile (12) comprend un élément central ayant une dureté Shore A supérieure ou égale à 80.

10. Endoscope (1) selon la revendication 9, dans lequel l'élément central est recouvert d'un revêtement plus mou.

11. Endoscope (1) selon la revendication 10, dans lequel le revêtement a une dureté Shore A dans la plage de 40 à 60, de préférence approximativement 50.

12. Système comprenant un dispositif d'affichage (100) et un endoscope (1) selon l'une quelconque des revendications précédentes, pouvant être connecté audit dispositif d'affichage (100).
